# EUROPEAN PATENT SPECIFICATION

(11) **EP 3 424 348 B1**
(45) Date of publication and mention of the grant of the patent: **18.12.2019**
(21) Application number: 17850078.1
(22) Date of filing: 04.07.2017
(51) Int. Cl.: A23L 33/00, A23L 13/20, A23L 19/00, A23L 5/20, A23L 13/00, A61K 35/407, A22C 17/08, A23B 4/24

(54) **PROCESSING METHOD FOR DETOXIFIED ANIMAL LIVER FOODSTUFF SUITABLE FOR INFANTS**
VERARBEITUNGSVERFAHREN FÜR LEBENSMITTEL AUS ENTGIFTETER TIERLEBER FÜR KLEINKINDER
PROCÉDÉ DE TRAITEMENT DE PRODUIT ALIMENTAIRE À BASE DE FOIE ANIMAL DÉTOXIFIÉ APPROPRIÉ POUR DES NOURRISSONS

(30) Priority: 10.04.2017 CN 201710228702
(43) Date of publication of application: 09.01.2019
(73) Proprietor: Qingdao University, Qingdao, Shandong 266000 (CN)
(72) Inventor: DONG, Qian, Qingdao Shandong 266000 (CN); YU, Qiyue, Qingdao Shandong 266000 (CN); WEI, Bin, Qingdao Shandong 266000 (CN); XIA, Nan, Qingdao Shandong 266000 (CN); DONG, Bingzi, Qingdao Shandong 266000 (CN); ZHU, Chengzhan, Qingdao Shandong 266000 (CN)
(74) Representative: J A Kemp LLP
(86) International application number: PCT/CN2017/091685
(87) International publication number: WO 2018/049875

(56) References cited:
- WO-A1-2015/138832
- CN-A- 106 954 804
- CN-A- 106 962 804
- CN-U- 202 050 840
- CN-U- 202 958 590
- US-A- 1 557 053
- US-A- 2 471 282
- US-A- 5 464 638
- US-A1- 2010 092 939
- US-A1- 2015 181 901
- Chelsea Thiede: "Grass-fed Beef, Liver, and Carrot Puree (Weston A Price/Paleo First Baby Foods) - Fed and Focused", , 11 November 2016 (2016-11-11), XP055583677, Retrieved from the Internet: URL:https://www.fedandfocused.com/baby-foo ds/beef-liver-and-carrot-puree-weston-a-pr icepaleo-first-baby-foods/ [retrieved on 2019-04-26]
- MENG, FEI: "Steamed Mashed Liver, Full Guidance for Gestation, Delivery and Parenting", , 31 December 2014 (2014-12-31), page 662,
- XU, BOLIANG et al.: "Points for Attention in Eating Animal Livers, Food Contamination and Food Safety", , 30 September 1992 (1992-09-30), pages 216-217, XP009510351,
- FAN, ZHENJIANG et al.: "Meat Product Processing Technology, Food Processing Technology", , 28 February 2013 (2013-02-28), page 25, XP009510350, ISBN: 9787504663108

## Description

### TECHNICAL FIELD

The present application relates to the field of food products, and in particular to detoxified animal liver food products suitable for infants and processing methods thereof.

### BACKGROUND OF THE RELATED ART

The animal liver, e.g. pork liver, which is more than 10 times greater in nutrient content than that of pork, is highly nutritional. The content of vitamin A in the animal liver is greater than that in other food products such as milk, eggs, meats and fish. Vitamin A can protect eyes, maintain normal eyesight, prevent dry eyes and eye fatigue, help the bones development of infants, promote the restoration of epidermal tissues, and have therapeutic effect on night blindness. In addition, the animal liver also contains vitamin B2 which can maintain healthy complexion and normal functions of growth and reproduction, enhance human body's immune reaction, and have functions of anti-oxidation and anti-aging. The animal liver further contains rich proteins and animal iron, which is a kind of preferred nutritious food product for children with nutritional anemia. However, the animal liver is also the largest toxicants transfer station and the detoxifying organ within animal body. Most of toxicants in the blood, even toxicants and pathogens combined with proteins, can enter into the liver. In today's increasingly serious pollution of the environment, the content of toxins and pathogens in the animal liver is many times higher than that in muscle, and a large number of macrophages and immune cells are present in hepatic sinusoids of the liver, which phagocytose toxins and pathogens in the liver. These macrophages and immune cells containing a large number of toxins and pathogens aggregate in tens of thousands of hepatic sinusoids in the liver. If these macrophages and immune cells containing a large number of toxins and pathogens are not removed, it is impossible to achieve complete detoxification of liver food products.

In the previous methods of detoxifying the animal liver, the liver is firstly cut into pieces, and these pieces are washed with tap water for 10 minutes and then soaked in water for 30 minutes. However, due to the effect that the blood would be rapidly coagulated after the liver is separated from the animal body and macrophages and other immune cells containing a large number of toxins in the blood may be aggregated in tens of thousands of hepatic sinusoids in the liver with blood coagulation, only a small amount of toxins and harmful substances contained in the liver may be removed, and most of toxins and harmful substances may still remain in the hepatic sinusoids and micrangium in the liver, even if the liver is cut into pieces which are then washed and soaked. Therefore, the traditional methods of detoxifying the animal liver cannot achieve complete detoxification.

It has been proposed in the feeding recommendation for 0 to 3-year-old infants that the introduced foods should be mainly iron-fortified foods, in which the animal liver is a good iron source. However, since the liver is a detoxifying organ, some toxic substances are easily accumulated therein. Therefore, it is not recommended adding too much animal liver into food products.

In other words, due to the following two reasons, namely the animal liver contains a large number of toxins and the conventional processing methods cannot completely remove the toxins from the animal liver, the infant's intake of the animal liver is limited, and further, the development of animal liver food products for infants is also limited.

US1557053A discloses a new food product comprising an animal liver in finely divided condition.

Chelsea Thiede:" Grass-fed Beef, Liver, and Carrot Puree (Weston A Price/Paleo First Baby Foods) - Fed and Focused", 11 November 2016, discloses a homemade baby food recipe that mixing liver with grass-fed beef and sweet root vegetables.

US2471282A discloses a process for conditioning the blood vessels of an animal liver with a proteolytic enzyme for the tenderization thereof without affecting the meat tissue of the liver.

US 2015/181901A discloses methods for tissue storage.

US 5464638A discloses a process for tenderizing, flavoring, cholesterol or sodium extracting or fat binding of meat through perfusion.

### SUMMARY OF THE INVENTION

According to the above deficiencies, an object of the present application is to provide detoxified animal liver food products suitable for infants and processing methods thereof. Detoxified animal liver is made into liver paste which is then processed and packaged in cans. Such food products can be served immediately and thus it is very convenient.

In order to achieve the above object, the technical scheme of the present application is to provide a processing method of a detoxified animal liver food product suitable for infants, wherein the method comprises the following steps:
S1: taking out a whole liver from an animal body and removing gallbladder, with blood vessels in first porta hepatis of the liver being retained when taking out the liver;
S2: spreading the liver taken out on a clean plane, dissecting the first porta hepatis of the liver and determining portal vein, biliary tract, hepatic artery and upper and lower ends of the inferior vena cava, and then inserting disinfected medical catheters into blood vessels of the portal vein, the biliary tract and blood vessels of the hepatic artery, respectively;
S3: perfusing a perfusate of 30°C-35°C into the catheters in the portal vein, the hepatic artery and the biliary tract, the perfusate fully flows into a hepatic sinusoid system through the portal vein and the hepatic artery and fully flows into a biliary tract system through the biliary tract;
S4: perfusing the perfusate continuously, macrophages and other immune cells containing a large number of toxins, blood and harmful substances in the liver are eliminated from the upper and lower ends of the inferior vena cava continuously and bile is flushed out from the biliary tract system;
S5: perfusing continuously, until the liver becomes white from blood red, and then taking down the catheters from the portal vein, the biliary tract and the hepatic artery of the liver to complete the vascular perfusion cleaning, detoxification and cells and tissues preservation of the liver;
   the animal liver is pork liver, beef liver or lamb liver;
S6: configuring materials: mixing starch uniformly with water for standby;
S7: eliminating the perfusate from the animal liver;
S8: adding carrots into the animal liver from which the perfusate is eliminated, grinding into a homogenate, adding the uniformly mixed starch into the homogenate, mixing uniformly once again, and then cooking;
S9: adding nutritional additives into the cooked animal liver homogenate uniformly after the cooked animal liver homogenate is cooled, then filling in cans, vacuuming, sealing, disinfecting, cooling and packaging.

Preferably, the length of the blood vessels retained in the first porta hepatis is 3 cm to 4 cm.

Preferably, in step S1, ensuring integrity of surface capsula of the liver taken out in step S1.

Preferably, in step S2,fixing joints where the blood vessels of the portal vein, the biliary tract and the blood vessels of the hepatic artery connect with the catheters.

Preferably, in step S3, the perfusion speed of the perfusate is 20 ml/s to 30 ml/s.

Preferably, in step S3, the perfusate is a solution of water and salt, the ratio of the salt to the water is 8.5-9 grams of the salt per thousand milliliters of the water.

Preferably, in step S3, the perfusate is a solution of water and sodium chloride, i.e. an aqueous solution of sodium chloride, the ratio of the sodium chloride to the water is 8.5-9 grams of the sodium chloride per thousand milliliters of the water.

The beneficial effects of the present application are the followings: in the present application, the food products suitable for infants are made by using the detoxified animal liver, the liver paste contains rich nutrients such as protein, iron, Va, Vb2, it is non-toxic, odorless and safe to serve. Such liver paste provides infants with a good natural nutritional supplement and offers a new development opportunity for animal liver food products.

### BRIEF DESCRIPTION OF THE DRAWINGS

Fig. 1 is a micrograph of the morphology of liver tissues after being vascular perfusion cleaned according to Embodiment 1 (4x objective);
Fig. 2 is a micrograph of the morphology of liver tissues without vascular perfusion cleaning (4x objective);
Fig. 3 is a micrograph of the morphology of liver tissues after being vascular perfusion cleaned according to Embodiment 1 (10x objective);
Fig. 4 is a micrograph of the morphology of liver tissues without vascular perfusion cleaning (10x objective);
Fig. 5 is a micrograph of the morphology of liver tissues after being vascular perfusion cleaned according to Embodiment 1 (20x objective);
Fig. 6 is a micrograph of the morphology of liver tissues without vascular perfusion cleaning (20x objective);
Fig. 7 is a micrograph of the morphology of liver tissues after being vascular perfusion cleaned according to Embodiment 1 (40x objective);
Fig. 8 is a micrograph of the morphology of liver tissues without vascular perfusion cleaning (40x objective).

### DETAILED DESCRIPTION OF THE INVENTION

The invention is as defined in the appended claims. The present disclosure will be further described below in conjunction with specific embodiments.

The present disclosure relates to a detoxified animal liver food product suitable for infants, wherein the detoxified animal liver is obtained according to the method as below:
S1: Taking out a whole fresh liver quickly from an animal body, removing gallbladder, with blood vessels in first porta hepatis of the liver being retained when taking out the liver. The propose of retaining the blood vessels in the first porta hepatis of the liver is to make it easier to determine the blood vessels during the subsequent dissection and perfusion and insert catheters into the blood vessels quickly.
S2: Spreading the liver taken out gently on a clean plane, dissecting the first porta hepatis of the liver quickly and determining portal vein, biliary tract, hepatic artery and upper and lower ends of the inferior vena cava, and then inserting disinfected medical catheters into blood vessels of the portal vein, the biliary tract and blood vessels of the hepatic artery, respectively. Wherein, the upper and lower ends of the inferior vena cava are not limited to upper and lower ends and may also be represented as two ends of the inferior vena cava.
S3: Perfusing a perfusate of 30°C to 35°C into the catheters in the portal vein, the hepatic artery and the biliary tract, the perfusate fully flows into a hepatic sinusoid system through the portal vein and the hepatic artery and fully flows into a biliary tract system through the biliary tract. Warm perfusate can contribute to a successful elimination of macrophages and other immune cells containing a large number of toxins, blood and harmful substances and bitter bile in the liver. If the temperature of the perfusate is too low, the blood in the hepatic sinusoid would coagulate quickly, which is unfavourable for the perfusion process. If the temperature of the perfusate is too high, it is easy to destroy cells and tissues of the liver. Therefore, the temperature of the perfusate is selected as a temperature which is close to the animal's body temperature for perfusion. It can be understood that the temperature of the perfusate can be rationally selected within the above range, for example, it can be 32°C, 33°C, 34°C, etc.

The processes of slaughtering animals, taking out the liver and the perfusion should be performed quickly. If it is failed to perform the perfusion within a very short period of time, the blood in the hepatic sinusoid of the liver would coagulate quickly, and the internal liver tissues and immune cell structure would be autolyzed and destroyed. Therefore, in order to ensure the freshness of the liver, the perfusion must be performed within a very short period of time to keep the liver tissue structure from being destroyed. During the actual operation, it takes within ca.10 minutes.

S4: Perfusing the perfusate continuously, macrophages and other immune cells containing a large number of toxins, blood and harmful substances in the liver are eliminated from the upper and lower ends of the inferior vena cava continuously and bile is flushed out from the biliary tract system. By perfusing and washing the hepatic sinusoid system and biliary tract system of the liver respectively, macrophages and other immune cells containing a large number of toxins, blood and harmful substances, as well as bile are removed. This guarantees the liver food product safety, and removes bitterness of the liver food product.

S5: Perfusing continuously, until the fresh liver becomes white from blood red, and then taking down the catheters from the portal vein, the biliary tract and the hepatic artery of the liver to complete the vascular perfusion cleaning, detoxification and cells and tissues preservation of the liver.

By perfusing the blood vessels and the hepatic sinusoids in the liver with the perfusate of a temperature close to the animal's body temperature quickly after the animal liver being separated, macrophages and other immune cells containing a large number of toxins, blood and harmful substances in the liver are eliminated continually from the upper and lower ends of the inferior vena cava, and bitter bile is continually eliminated through the biliary tract, the above-mentioned detoxified animal liver used in the detoxified animal liver food product suitable for infants achieves vascular perfusion cleaning and detoxifying to the liver. As for the processed animal liver, it can ensure, to a great extent, that the liver cells and tissues keep away from being destroyed as far as possible, and macrophages and other immune cells containing a large number of toxins, blood and harmful substances in the liver can be removed, which provides a healthy safeguard for the consumption of the liver.

As a preferred implementation, a length of the blood vessels retained in the first porta hepatis is 3 cm to 4 cm. This aims at a better insertion of the catheters into the blood vessels and is helpful for fixation of the blood vessels with the catheters. If the blood vessels retained are too short, firstly, it is time-consuming to find them; secondly, it is not conducive to fix them with the catheters. Therefore, the blood vessels retained should be a little bit long, for example, the length of the above-mentioned blood vessels retained can be 3.2 cm, 3.4 cm, 3.6 cm, 3.8 cm, etc.

As a preferred implementation, in step S1, ensuring integrity of surface capsula of the liver taken out in step S1. If the surface capsula of the liver is broken during the operation, the perfusate used in the sequent perfusion would flow out from the broken surface capsula of the liver, which may cause incomplete perfusion cleaning as well as incomplete detoxification to the vessels of the liver.

As a preferred implementation, in step S2, fixing joints where the blood vessels of the portal vein, the biliary tract, the blood vessels of the hepatic artery connect with the catheters, in order to prevent the perfusate from flowing out from gaps between the blood vessels and the catheters, which causes incomplete perfusion cleaning and detoxification. The above fixation can be achieved by selecting conventional tools such as ropes and clamps etc. In addition, other methods for fixing the blood vessels with catheters can be used as alternates, as long as they conform to the food hygiene standards.

As a preferred implementation, in step 3, a perfusion speed of the perfusate is 20 ml/s to 30 ml/s. Such perfusion speed can enhance the efficiency and effectiveness of the perfusion and of the elimination of the toxins, the blood and the harmful substances, and can protect the liver cells and tissues from being destroyed to a maximum extent. The perfusion speed can be rationally selected within the above range as required, for example, it can be 22 ml/s, 24 ml/s, 25 ml/s, 26 ml/s, 28 ml/s, etc.

As a preferred implementation, in step S3, the perfusate is a solution of water and salt, a ratio of the salt to the water is 8.5 to 9 grams of the salt per 1000 milliliters of the water. The concentration of the perfusate can be rationally selected within the above range, for example, 8.6g, 8.7g, 8.8g, 8.9g, etc of the salt per 1000 milliliters of the water.

As a preferred implementation, in step S3, the perfusate is a solution of water and salt, i.e. an aqueous solution of sodium chloride, the ratio of the sodium chloride to the water is 8.5-9 grams of the sodium chloride per 1000 milliliters of the water. It can be understood that the concentration of the perfusate can be rationally selected within the above range, for example, 8.6g, 8.7g, 8.8g, 8.9g, etc of the sodium chloride per 1000 milliliters of the water.

As a preferred implementation, the animal liver used in the present application comprises, but is not limited to, pork liver, beef liver and lamb liver.

As a preferred implementation, the above-mentioned detoxified animal liver food product suitable for infants further comprises starch, carrots and nutritional additives.

As a preferred implementation, the above-mentioned detoxified animal liver food product further comprises one or more of starch, carrots and nutritional additives. As a preferred implementation, the nutritional additives are natural nutritional additives.

Carrot is a crisp, delicious and nutrient-rich common vegetable, known as "small ginseng". Carrot contains rich nutrient ingredients such as sugar, fat, volatile oil, carotene, vitamin A, vitamin B1, vitamin B2, anthocyanin, calcium, iron, which is also very suitable for infant's consumption. However, it can be also understood that carrot can be replaced with other food materials suitable for infants. Such food materials can be for example, one or more of fruits, such as apples, watermelons, oranges and kiwi fruit, which contain rich vitamins and nutrients. In addition, the nutritional additives can be natural nutritional additives suitable for infants such as DHA, vitamins and minerals etc. The proportion of the added nutritional additives can be properly adjusted according to the demand for nutrients for infants of different months of age.

The present disclosure further provides a processing method of the above-mentioned detoxified animal liver food product suitable for infants. The method comprises the following steps:
S1. configuring materials: mixing starch and nutritional additives uniformly with water for standby;
S2. eliminating the perfusate from the detoxified animal liver in the above-mentioned detoxified animal liver food product suitable for infants;
S3. adding carrots into the animal liver from which the perfusate is eliminated, grinding into a homogenate, adding the uniformly mixed starch and nutritional additives into the homogenate, and then mixing uniformly once again for standby;
S4. cooking the animal liver homogenate obtained in step S3, and then filling in cans, vacuumizing, sealing, disinfecting, cooling and packaging to obtain a canned and instant detoxified animal liver paste food product suitable for infants.

In case that the nutritional additives are susceptible to deterioration or ineffectiveness when being heated, they can be added after the animal liver is cooked and cooled to ensure the effectiveness of the nutritional additives.

For nutritional additives that cannot withstand high temperature, the present application further provides a processing method of the above-mentioned detoxified animal liver food product suitable for infants. The method comprises the following steps:
S1. configuring materials: mixing starch uniformly with water for standby;
S2. eliminating the perfusate from the detoxified animal liver of the above-mentioned detoxified animal liver food product suitable for infants;
S3. adding carrots into the animal liver from which the perfusate is eliminated, grinding into a homogenate, adding the uniformly mixed starch into the homogenate, mixing uniformly once again, and then cooking;
S4. adding nutritional additives into the cooked animal liver homogenate uniformly after the cooked animal liver homogenate is cooled, then filling in cans, vacuumizing, sealing, disinfecting, cooling and packaging to obtain a canned and instant detoxified animal liver paste food product suitable for infants.

Since the masticatory function of 0 to 3-year-old infants has not been fully developed, it is conductive to infant's consumption and digestion by grinding the animal liver into the homogenate. Certainly, the animal liver can be grinded into a homogenate with different particle sizes according to different months of age and different development states of the teeth or of the mastication of infants. For example, the particle size of the homogenate of animal liver should be as small as possible for consumption of infants within 1 year old. For children between 1 year old and 2 years old who have grown teeth and have certain masticatory ability, the particle size of the homogenate can be properly increased, which is helpful to train the masticatory function of their teeth. For children over 2 years old whose masticatory function has been developed well, the particle size of the homogenate can be further increased. For older children, the animal liver can be cut into particles of about 1 centimeter.

The detoxified animal liver is made into liver paste, packaged in cans etc. The liver paste is rich in nutrients and without preservatives, which is ideal for infant's consumption. Such canned food product is instant after removing the package, so it is very convenient. The liver paste can be packaged quantitatively and independently according to the amount of the infant's consumption for each time. When being consumed, the liver pastes in these quantitative and independent packages do not affect each other, thereby ensuring that the liver paste is fresh and protect it from contamination.

There is no strict limit on the nutritional additives as well. Such nutritional additives can be properly added according to the development characteristics of infants. For example, in order to facilitate the development of the eyesight and the nervous system of infants, a certain amount of DHA can be properly added. If the gastrointestinal function of infants is weak, a certain amount of probiotics can be added. In addition, for infants in different situations, enhanced nutritional additives can be added intentionally.

In the blood circulation system of the liver, the portal vein is the functional vessel of the liver, which delivers substances from the stomach and intestine into the liver. The portal vein is divided into left and right branches at the porta hepatis, which run to left and right lobes of the liver, respectively, and are then repeatedly branched between the hepatic lobules to form interlobular veins. Interlobular veins are branched into small branches, described as terminal portal venules, which run between two adjacent hepatic lobules. Branches of the terminal portal venules are connected to the hepatic sinusoids to deliver the portal vein blood into the hepatic lobules. In addition, the hepatic arterial blood is rich in oxygen and the hepatic artery is the nutritional vessel of the liver. Branches of the hepatic artery, which are accompanied with branches of the portal vein, are branched into interlobular arteries, and the interlobular arteries are then branched into terminal hepatic arterioles that finally run into the hepatic sinusoids. Interlobular arteries are also branched into small branches to supply the capsula, mesenchyme and bile ducts. Therefore, the hepatic sinusoids contain mixed blood from the portal veins and the hepatic arteries. The blood in the hepatic sinusoids flows from the periphery of the lobules to the center and enters into central veins. There is no smooth muscle outside the endothelium of the central veins, and instead, only a few of connective tissues are present therein. Several central veins merge into sublobular veins. The sublobular veins run in interlobular connective tissues separately and have a large diameter and a large thickness. The sublobular veins further merge into 2 or 3 hepatic veins, which then run into the inferior vena cava after exit from the liver.

It can be known according to the physiological anatomic structure of the liver that, by perfusing the perfusate through the portal vein, biliary tract and hepatic artery of the liver and by eliminating macrophages and other immune cells containing a large number of toxins, blood and harmful substances in the liver through the upper and lower ends of the inferior vena cava, toxins, blood and harmful substances in all parts of the liver can be eliminated completely.

The animal liver used in the present application comprises, but is not limited to, pork liver, beef liver and lamb liver.

The present application will be further described below by specific embodiments.

### Embodiment 1

A detoxified animal liver food product suitable for infants is provided, wherein the detoxified animal liver was obtained according to the method as below:
S1: Taking out the whole fresh pork liver quickly from a pig body and removing gallbladder, with blood vessels in first porta hepatis of the pork liver being retained when taking out the pork liver; ensuring the integrity of surface capsula of the pork liver taken out; and retaining the length of the blood vessels in the first porta hepatis with 3 cm.
S2: Spreading the pork liver taken out gently on a clean plane, dissecting the first porta hepatis of the pork liver quickly and determining the portal vein, biliary tract, hepatic artery and the upper and lower ends of the inferior vena cava, inserting disinfected medical catheters into blood vessels of the portal vein, the biliary tract and blood vessels of the hepatic artery, respectively; and fixing the joints where the blood vessels of the portal vein, the biliary tract, the blood vessels of the hepatic artery connect with the catheters.
S3: Perfusing a perfusate of 30°C into the catheters in the portal vein, the hepatic artery and the biliary tract, the perfusate fully flows into a hepatic sinusoid system through the portal vein and the hepatic artery and fully flows into a biliary tract system through the biliary tract. The perfusion speed of the perfusate was 20 ml/s. The perfusate was a solution of water and salt, the ratio of the salt to the water was 8.5 grams of the salt per 1000 milliliters of the water.
S4: Perfusing the perfusate continuously, macrophages and other immune cells containing a large number of toxins, blood and harmful substances in the liver were eliminated from the upper and lower ends of the inferior vena cava continuously, and bile was flushed out from the biliary tract system.
S5: Perfusing continuously, until the fresh pork liver became white from blood red, and then taking down the catheters from the portal vein, the biliary tract and the hepatic artery of the pork liver to complete the vascular perfusion cleaning, detoxification and cells and tissues preservation of the pork liver and to obtain the detoxified pork liver.

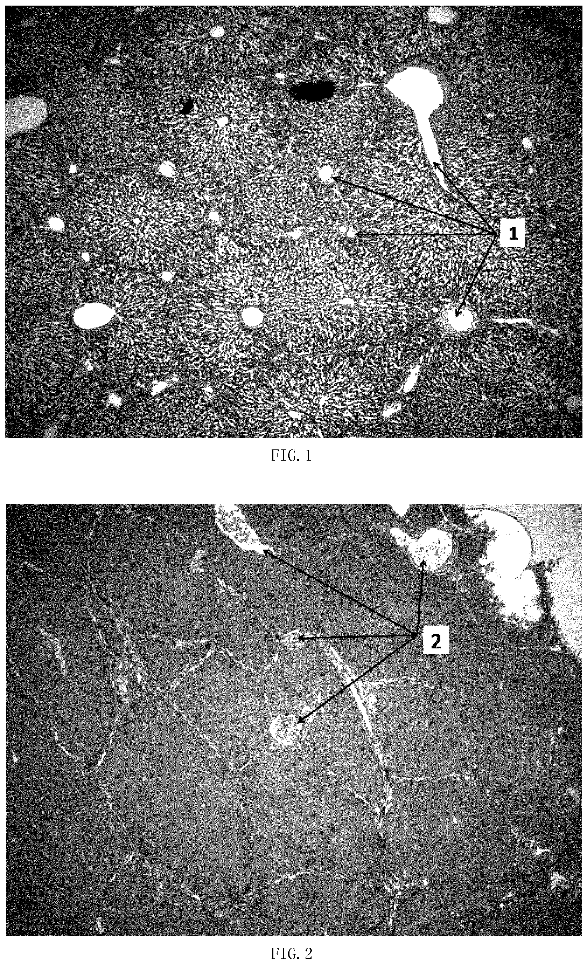

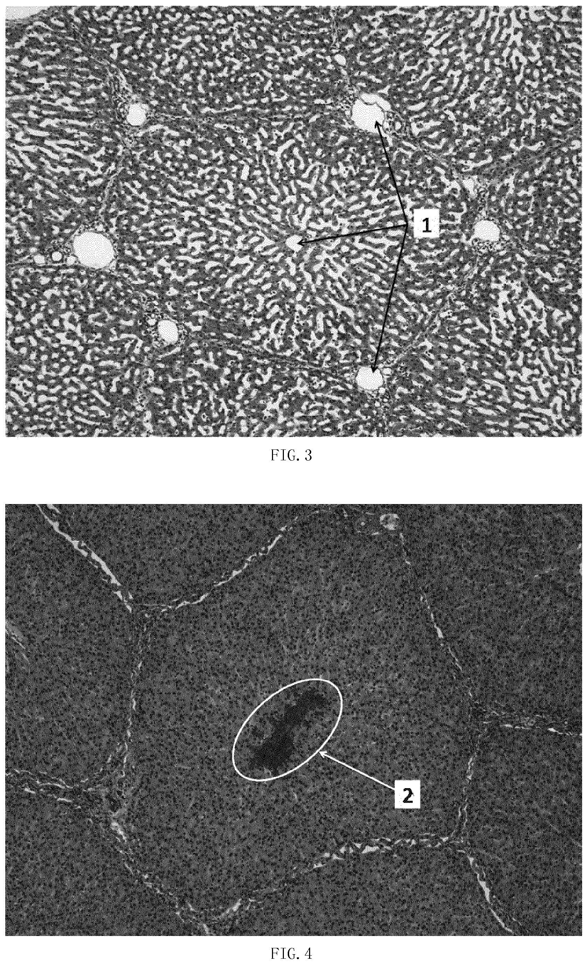

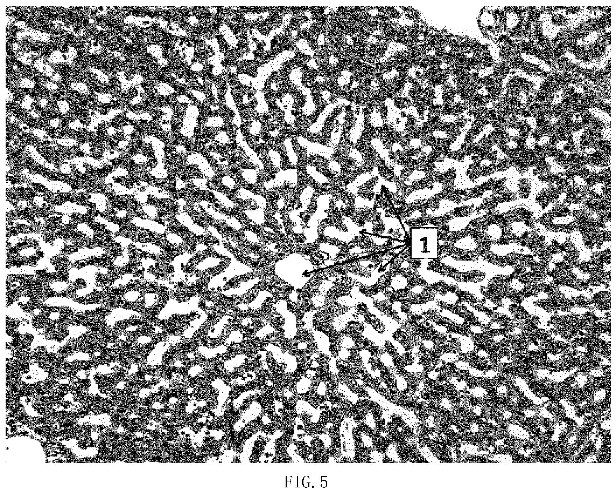

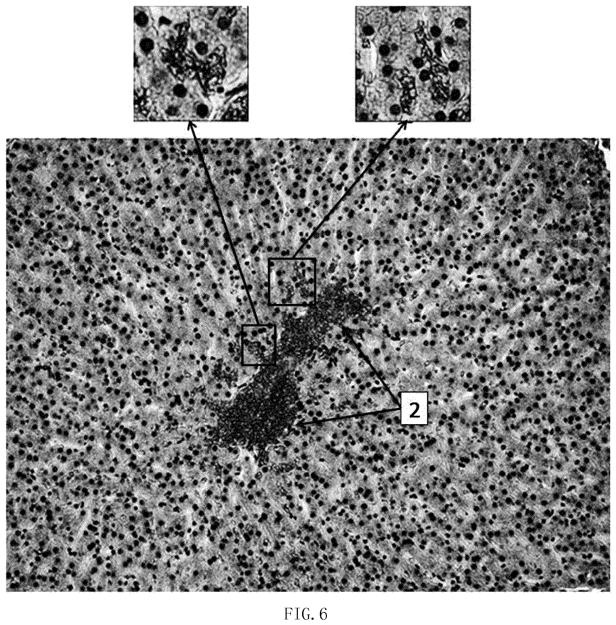

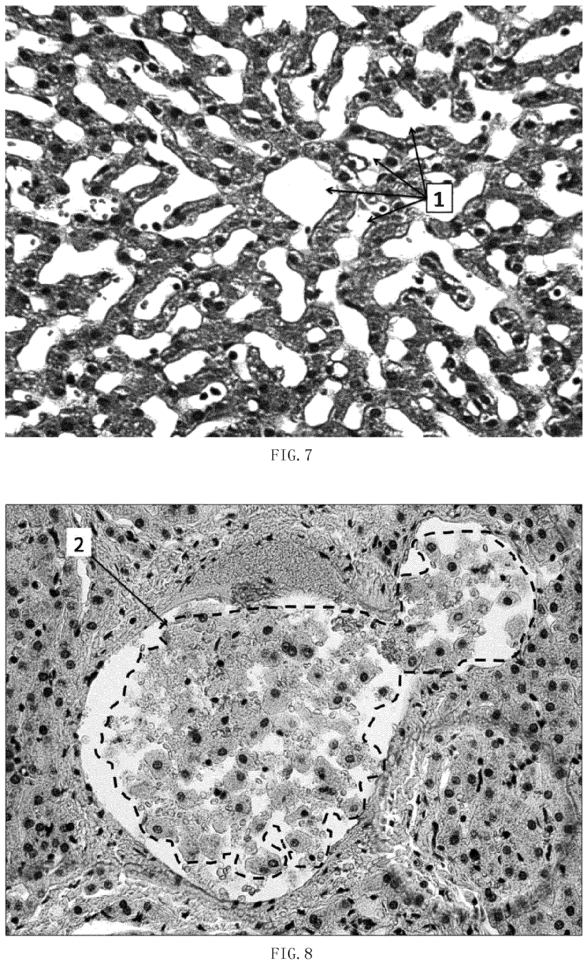

Figs. 1 to 8 are micrographs of pork liver tissues of this embodiment after being vascular perfusion cleaned at different magnifications and micrographs of liver tissues without any processing at corresponding magnifications. Wherein, in the morphology of liver tissues after being cleaned by the method of this embodiment, 1 represents a blank area left after eliminating original macrophages and immune cells phagocytosing toxins and containing pathogens by vascular perfusion, and 2 represents macrophages and immune cells phagocytosing toxins and containing pathogens contained in liver tissues without processing. It should be noted that 1 and 2 in the drawings are exemplarily marked, not exhaustive.

It can be known from comparison and analysis of the above micrographs that, in the animal liver obtained by cleaning and detoxifying by the method of this embodiment, toxic tissue cells such as macrophages and immune cells phagocytosing toxins and containing pathogens are basically not found even if at high magnifications. It means that macrophages and immune cells phagocytosing toxins and containing pathogens contained in the liver tissues are removed basically, and the cleaning and detoxification of liver tissues are completed. In addition, the characteristics of liver cells are obvious, almost not destroyed. Such animal liver provides raw material for the subsequent preparation of the detoxified animal liver food products suitable for infants.

The detoxified animal liver food product suitable for infants according to this embodiment comprises the above-mentioned fresh detoxified pork liver, and further comprises starch, carrots and nutritional additives.

This embodiment further provides a processing method of the above-mentioned detoxified animal liver food product suitable for infants. The method comprised the following steps:
S1. configuring materials: mixing starch and nutritional additives uniformly with water for standby;
S2. eliminating the perfusate from the above-mentioned detoxified pork liver;
S3. adding carrots into the pork liver from which the perfusate is eliminated, grinding into a homogenate, adding the uniformly mixed starch and nutritional additives into the homogenate and mixing uniformly once again for standby; wherein the nutritional additives were a proper amount of Va, Vb, calcium and magnesium, etc.;
S4. cooking the pork liver homogenate made in step S3, and then filling it in cans, vacuumizing, sealing, disinfecting, cooling and packaging to obtain a canned and instant detoxified animal liver paste food product suitable for infants.

The liver paste was quantitatively filled in cans according to infants' months of age.

### Embodiment 2

A detoxified animal liver food product suitable for infants is provided, wherein the detoxified animal liver was obtained according to the method as below:
S1: Taking out the whole fresh beef liver quickly from a cattle body and removing gallbladder, with vessels in first porta hepatis of the liver being retained when taking out the beef liver; ensuring the integrity of epidermis of the beef liver taken out; retaining the length of the blood vessels in the first porta hepatis with 4 cm.
S2: Spreading the beef liver taken out gently on a clean plane, dissecting the first porta hepatis of the beef liver quickly and determining the portal vein, biliary tract, hepatic artery and the upper and lower ends of the inferior vena cava, inserting disinfected medical catheters into blood vessels of the portal vein, the biliary tract and blood vessels of the hepatic artery, respectively; fixing the joints where the blood vessels of the portal vein, the biliary tract, the blood vessels of the hepatic artery connect with the catheters.
S3: Perfusing a perfusate of 35°C into the catheters in the portal vein, the hepatic artery and the biliary tract, the perfusate fully flows into a hepatic sinusoid system through the portal vein and the hepatic artery and fully flows into a biliary tract system through the biliary tract. The perfusion speed of the perfusate is was 30 ml/s. The perfusate was a solution of water and salt, the ratio of the salt to the water was 9 grams of the salt per 1000 milliliters of the water.
S4: Perfusing the perfusate continuously, macrophages and other immune cells containing a large number of toxins, blood and harmful substances in the liver were eliminated from the upper and lower ends of the inferior vena cava continuously and bile was flushed out from the biliary tract system.
S5: Perfusing continuously, until the fresh beef liver became white from blood red, and then taking down the catheters from the portal vein, the biliary tract and the hepatic artery of the liver to complete the vascular the perfusion cleaning, detoxification and cells and tissues preservation of the beef liver and to obtain the detoxified beef liver.

The micrograph of the morphology of the beef liver processed by the above method is similar to that of Figs. 1, 3, 5 and 7 in Embodiment 1. Here will be not described in details.

The detoxified animal liver food product suitable for infants provided by this embodiment comprises the above-mentioned fresh detoxified beef liver, and further comprises starch, carrots and nutritional additives.

This embodiment further provides a processing method of the above-mentioned detoxified animal liver food product suitable for infants, The method comprised the following steps:
S1. configuring the materials: mixing starch uniformly with water for standby;
S2. eliminating the perfusate from the above-mentioned detoxified beef liver;
S3. adding carrots into the beef liver from which the perfusate is eliminated, grinding them into a homogenate, adding the uniformly mixed starch into the homogenate, mixing them uniformly once again and then cooking;
S4. adding nutritional additives into the cooked animal beef liver homogenate uniformly after the cooked animal beef liver homogenate was cooled, then filling it in cans, vacuumizing, sealing, disinfecting, cooling and packaging to obtain a canned and instant detoxified animal liver paste food product suitable for infants;
   Wherein, the nutritional additives were a proper amount of DHA and probiotics.

The liver paste was quantitatively filled in cans according to infants' months of age.

### Embodiment 3

A detoxified animal liver food product suitable for infants is provided, wherein the detoxified animal liver was obtained according to the method as below:
S1: Taking out the whole fresh lamb liver quickly from a lamb body and removing gallbladder, with blood vessels in first porta hepatis of the lamb liver being retained when taking out the lamb liver; ensuring the integrity of surface capsula of the lamb liver taken out; and retaining the length of the blood vessels in the first porta hepatis with 4 cm.
S2: Spreading the lamb liver taken out gently on a clean plane, dissecting the first porta hepatis of the lamb liver quickly and determining the portal vein, biliary tract, hepatic artery and the upper and lower ends of the inferior vena cava, inserting disinfected medical catheters into blood vessels of the portal vein, the biliary tract and blood vessels of the hepatic artery, respectively; and fixing the joints where the blood vessels of the portal vein, the biliary tract, the blood vessels of the hepatic artery connect with the catheters.
S3: Perfusing a perfusate of 33°C into the catheters in the portal vein, the hepatic artery and the biliary tract, the perfusate fully flows into a hepatic sinusoid system through the portal vein and the hepatic artery and fully flows into a biliary tract system through the biliary tract. The perfusion speed of the perfusate was 25 ml/s. The perfusate was a solution of water and salt, the ratio of the salt to the water was 9 grams of the salt per 1000 milliliters of the water.
S4: Perfusing the perfusate continuously, macrophages and other immune cells containing a large number of toxins, blood and harmful substances in the liver were eliminated from the upper and lower ends of the inferior vena cava continuously, and bile was flushed out from the biliary tract system.
S5: perfusing continuously, until the fresh lamb liver became white from blood red, and then taking down the catheters from the portal vein, the biliary tract and the hepatic artery of the lamb liver to complete the vascular perfusion cleaning, detoxification and cells and tissues preservation of the lamb liver and to obtain the detoxified lamb liver.

The micrograph of the morphology of the lamb liver processed by the above method is similar to that of Figs. 1, 3, 5 and 7 in Embodiment 1. Here will be not described in details.

The detoxified animal liver food product suitable for infants provided by this embodiment comprises the above-mentioned fresh detoxified lamb liver, and further comprises starch, carrots and nutritional additives.

This embodiment further provides a processing method of the above-mentioned detoxified animal liver food product suitable for infants. The method comprised the following steps:
S1. configuring materials: mixing starch and nutritional additives uniformly with water for standby;
S2. taking out the detoxified lamb liver from the perfusate, untying the ligated portal vein, biliary tract, hepatic artery and upper and lower ends of the inferior vena cava to eliminate the perfusate from the lamb liver;
S3. adding carrots into the lamb liver from which the perfusate is eliminated, grinding them into a homogenate, adding the uniformly mixed starch and nutritional additives into the homogenate and mixing them uniformly once again for standby, wherein the nutritional additives were calcium, magnesium and zinc;
S4. cooking the lamb liver homogenate made in step S3, and then filling it in cans, vacuumizing, sealing, disinfecting, cooling and packaging to obtain a canned and instant detoxified animal liver paste food product suitable for infants.

The liver paste was quantitatively filled in cans according to infants' months of age.

After being detoxified, the animal liver is made into liver paste which is then quantitatively filled in cans according to infants' months of age (i.e. the amount of consumption). The canned food products are instant after removing the packages, so it is very convenient. Such products supplement demand for iron requirement of infants and ensure that toxins in the animal liver will not cause secondary injuries to infants. It is safe to serve.

## Claims

1. A processing method of a detoxified animal liver food product suitable for infants, **characterized in that** the method comprises the following steps:
S1: taking out a whole liver from an animal body and removing gallbladder, with blood vessels in first porta hepatis of the liver being retained when taking out the liver;
S2: spreading the liver taken out on a clean plane, dissecting the first porta hepatis of the liver and determining portal vein, biliary tract, hepatic artery and upper and lower ends of inferior vena cava, and inserting disinfected medical catheters into blood vessels of the portal vein, the biliary tract and blood vessels of the hepatic artery;
S3: perfusing a perfusate of 30°C-35°C into the catheters in the portal vein, the hepatic artery and the biliary tract, the perfusate fully flows into a hepatic sinusoid system through the portal vein and the hepatic artery and fully flows into a biliary tract system through the biliary tract;
S4: perfusing the perfusate continuously, macrophages and other immune cells containing a large number of toxins, blood and harmful substances in the liver are eliminated from the upper and lower ends of the inferior vena cava continuously and bile is flushed out from the biliary tract system;
S5: perfusing continuously, until the liver becomes white from blood red, and then taking down the catheters from the portal vein, the biliary tract and the hepatic artery of the liver to complete the vascular perfusion cleaning, detoxification and cells and tissues preservation of the liver;
the animal liver is pork liver, beef liver or lamb liver;
S6: configuring materials: mixing starch uniformly with water for standby;
S7: eliminating the perfusate from the animal liver;
S8: adding carrots into the animal liver from which the perfusate is eliminated, grinding into a homogenate, adding the uniformly mixed starch into the homogenate, mixing uniformly once again, and then cooking;
S9: adding nutritional additives into the cooked animal liver homogenate uniformly after the cooked animal liver homogenate is cooled, then filling in cans, vacuuming, sealing, disinfecting, cooling and packaging.

2. The processing method of claim 1, **characterized in that** the length of the blood vessels retained in the first porta hepatis is 3 cm to 4 cm.

3. The processing method of claim 1, **characterized in that** in step S1, ensuring integrity of surface capsula of the liver taken out.

4. The processing method of claim 1, **characterized in that** in the step S2, fixing joints where the blood vessels of the portal vein, the biliary tract and the blood vessels of the hepatic artery connect with the catheters.

5. The processing method of claim 1, **characterized in that** in step S3, the perfusion speed of the perfusate is 20 ml/s to 30 ml/s.

6. The processing method of claim 1, **characterized in that** in step S3, the perfusate is a solution of water and salt, and the ratio of the salt to the water is 8.5-9 grams of the salt per thousand milliliters of the water.

7. The processing method of claim 1, **characterized in that**, in step S3, the perfusate is a solution of water and sodium chloride, namely an aqueous solution of sodium chloride, and the ratio of the sodium chloride to the water is 8.5-9 grams of the sodium chloride per thousand milliliters of the water.

## Patentansprüche

1. Verarbeitungsverfahren eines für Kleinkinder geeignetes entgiftetes Tierleberlebensmittelprodukts, **dadurch gekennzeichnet, dass** das Verfahren die folgenden Schritte umfasst:
S1: Herausnehmen einer ganzen Leber aus einem Tierkörper und Entfernen der Gallenblase, wobei die Blutgefäße in der ersten Porta hepatis der Leber beim Herausnehmen der Leber zurückbehalten werden;
S2: Ausbreiten der herausgenommenen Leber auf einer sauberen Fläche, Zerlegen Porta hepatis der Leber und Bestimmen der Portalvene, des Gallengangs, der Leberarterie und den oberen und unteren Enden der unteren Hohlvene, und Einführen von desinfizierten medizinischen Kathetern in die Blutgefäße der Portalvene, des Gallengangs und die Blutgefäße der Leberarterie;
S3: Perfundieren eines Perfusats von 30 °C bis 35 °C in die Katheter in die Portalvene, die Leberarterie und den Gallengang, wobei das Perfusat durch die Portalader und die Leberarterie vollständig in ein hepatisches Sinussystem fließt und durch den Gallengang vollständig in ein Gallengangsystem fließt;
S4: kontinuierliches Perfundieren des Perfusats, Makrophagen und andere Immunzellen, die eine große Anzahl von Toxinen, Blut und schädlichen Substanzen in der Leber enthalten, werden kontinuierlich von den oberen und unteren Enden der unteren Hohlvene beseitigt und Galle wird aus dem Gallengangsystem ausgeschwemmt;
S5: kontinuierliches Perfundieren, bis die Leber weiß von blutrot wird, und anschließendes Abnehmen der Katheter von der Portalvene, dem Gallengang und der Leberarterie, um die Gefäßperfusionsreinigung, die Entgiftung und den Erhalt von Zellen und Geweben der Leber zu vervollständigen;
wobei die Tierleber Schweine-, Rinder- oder Lammleber ist;
S6: Konfigurieren von Materialien: gleichmäßiges Mischen von Stärke mit Wasser für Bereitschaft;
S7: Beseitigen des Perfusats aus der Tierleber;
S8: Zugeben von Karotten in die Tierleber, aus der das Perfusat
beseitigt ist, Mahlen zu einem Homogenat, Zugeben der gleichmäßig gemischte Stärke in das Homogenat, nochmals gleichmäßiges Mischen und dann Kochen;
S9: gleichmäßiges Zugeben von Nahrungsergänzungsmitteln zum gekochten Tierleberhomogenat nach dem Abkühlen des gekochten Tierleberhomogenats, danach Abfüllen in Dosen, Absaugen, Versiegeln, Desinfizieren, Abkühlen und Verpacken.

2. Verarbeitungsverfahren nach Anspruch 1, **dadurch gekennzeichnet, dass** die Länge der Blutgefäße, die in der ersten Porta hepatis zurückgehalten werden, 3 cm bis 4 cm beträgt.

3. Verarbeitungsverfahren nach Anspruch 1, **dadurch gekennzeichnet, dass** in Schritt S1 die Unversehrtheit der Oberflächenkapsel der herausgenommenen Leber sichergestellt wird.

4. Verarbeitungsverfahren nach Anspruch 1, **dadurch gekennzeichnet, dass** in Schritt S2 Gelenke fixiert werden, wo die Blutgefäße der Portalvene, der Gallengang und die Blutgefäße der Leberarterie mit den Kathetern verbunden sind.

5. Verarbeitungsverfahren nach Anspruch 1, **dadurch gekennzeichnet, dass** in Schritt S3 die Perfusionsgeschwindigkeit des Perfusats 20 ml/s bis 30 ml/s beträgt.

6. Verarbeitungsverfahren nach Anspruch 1, **dadurch gekennzeichnet, dass** in Schritt S3 das Perfusat eine Lösung von Wasser und Salz ist und das Verhältnis von Salz zu Wasser 8,5 - 9 Gramm von Salz pro tausend Milliliter des Wassers beträgt.

7. Verarbeitungsverfahren nach Anspruch 1, **dadurch gekennzeichnet, dass** in Schritt S3 das Perfusat eine Lösung von Wasser und Natriumchlorid ist, nämlich eine wässrige Lösung von Natriumchlorid, und das J-Verhältnis von Natriumchlorid zu Wasser 8,5 - 9 Gramm von Natriumchlorid pro tausend Milliliter des Wassers beträgt.

## Revendications

1. Procédé de traitement d'un produit alimentaire à base de foie animal détoxiqué approprié pour le nourrisson, **caractérisé en ce que** le procédé comprend les étapes suivantes :
S1 : prélèvement d'un foie entier sur un corps animal et retrait de la vésicule biliaire, les vaisseaux sanguins dans le premier sillon transverse étant conservés lors du prélèvement du foie ;
S2 : étalement du foie prélevé sur un plan propre, dissection du premier sillon transverse du foie et détermination de la veine porte, des voies biliaires, de l'artère hépatique et des extrémités supérieure et inférieure de la veine cave inférieure, et insertions de cathéters médicaux désinfectés dans les vaisseaux sanguins de la veine porte, les voies biliaires et les vaisseaux sanguins de l'artère hépatiques ;
S3 : perfusion d'un perfusat de 30 °C à 35 °C dans les cathéters dans la veine porte, l'artère hépatique et les voies biliaires, le perfusat s'écoulant totalement dans un système sinusoïdal hépatique à travers la veine porte et l'artère hépatique et s'écoulant totalement dans un système de voies biliaires à travers les voies biliaires ;
S4 : perfusion du perfusat en continu, des macrophages et autres cellules immunitaires contenant un grand nombre de toxines, de sang et de substances nocives dans le foie étant éliminées des extrémités supérieure et inférieure de la veine cave inférieure en continu et la bile étant éliminée par rinçage du système de voies biliaires ;
S5 : perfusion en continu, jusqu'à ce que le foie passe du rouge sang au blanc, puis retrait des cathéters de la veine porte, des voies biliaires et de l'artère hépatique du foie pour achever le nettoyage par perfusion vasculaire, la détoxication et la préservation des cellules et tissu du foie ;
le foie d'animal étant du foie de porc, du foie de bœuf ou du foie d'agneau ;
S6 : configuration de matières : mélange uniforme d'amidon avec de l'eau pour mise en réserve ;
S7 : élimination du perfusat du foie d'animal ;
S8 : ajout de carottes au foie d'animal dont le perfusat est
éliminé, broyage en homogénat, ajout de l'amidon mélangé uniformément à l'homogénat, nouveau mélange uniforme, puis cuisson ;
S9 : ajout uniforme d'additifs nutritionnels à l'homogénat de foie animal cuit après refroidissement de l'homogénat de foie animal cuit, puis remplissage de boîtes, mise sous vide, sertissage, désinfection, refroidissement et conditionnement.

2. Procédé de traitement de la revendication 1, **caractérisé en ce que** la longueur des vaisseaux sanguins conservés dans le premier sillon transverse est de 3 cm à 4 cm.

3. Procédé de traitement de la revendication 1, **caractérisé en ce qu'**à l'étape S1, on s'assure de l'intégrité de la capsule de surface du foie prélevé.

4. Procédé de traitement de la revendication 1, **caractérisé en ce qu'**à l'étape S2, on fixe des jonctions là où les vaisseaux sanguins de la veine porte, les voies biliaires et les vaisseaux sanguins de l'artère hépatique sont reliés aux cathéters.

5. Procédé de traitement de la revendication 1, **caractérisé en ce qu'**à l'étape S3, la vitesse de perfusion du perfusat est de 20 ml/s à 30 ml/s.

6. Procédé de traitement de la revendication 1, **caractérisé en ce qu'**à l'étape S3, le perfusat est une solution d'eau et de sel, et le rapport entre le sel et l'eau est de 8,5 à 9 grammes de sel pour mille millilitres d'eau.

7. Procédé de traitement de la revendication 1, **caractérisé en ce qu'**à l'étape S3, le perfusat est une solution d'eau et de chlorure de sodium, à savoir une solution aqueuse de chlorure de sodium, et le rapport entre le chlorure de sodium et l'eau est de 8,5 à 9 grammes de chlorure de sodium pour mille millilitres d'eau.
